# EUROPÄISCHE PATENTANMELDUNG

(11) **EP 4 620 670 A1**
(43) Veröffentlichungstag der Anmeldung: **24.09.2025**
(21) Anmeldenummer: 24165661.0
(22) Anmeldetag: 22.03.2024
(51) Int. Cl.: B32B 27/08, B32B 5/02, B32B 7/022, B32B 27/12, B32B 27/16, B32B 27/20, B32B 27/30, B32B 27/32

(54) **ELASTISCHE FOLIE, NONWOVENELEMENT SOWIE HYGIENEARTIKEL**

(71) Anmelder: Nitto Advanced Film Gronau GmbH, 48599 Gronau (DE); Nitto Denko Corporation, Ibaraki-shi, Osaka 567-8680 (JP)
(72) Erfinder: SOLLMANN, Henner, 48599 Gronau (DE); SANDMANN, Nikolas, 48429 Rheine (DE); BLOOM, Sven, 48599 Gronau (DE)
(74) Vertreter: Andrejewski - Honke Patent- und Rechtsanwälte Partnerschaft mbB

(57) **Zusammenfassung**

Die Erfindung betrifft eine elastische Folie (3) mit einer elastischen Folienschicht (6) und zumindest einer ersten, mit der elastischen Folienschicht coextrudierten nicht-elastischen Deckschicht (4, 5), wobei die elastische Folienschicht (6) aus einer Polymermischung mit einer ersten Polymerkomponente A und einer zweite Polymerkomponente B gebildet ist und wobei die erste Polymerkomponente ein Polyolefin oder eine Polyolefinmischung ist. Erfindungsgemäß ist die zweite Polymerkomponente B ein Styrol-Block-Copolymer, welches in einer Menge zwischen 28 Gew.-% und 60 Gew.-% in der Polymermischung vorliegt und wobei die Polymermischung weniger als 1 Gew.-% inorganische und weniger als 5 Gew.-% organische Restbestandteile enthält.

## Beschreibung

Die Erfindung betrifft eine elastische Folie mit einer elastischen Folienschicht und zumindest einer ersten, mit der elastischen Folienschicht coextrudierten nicht-elastischen Deckschicht.

Eine derartige elastische Folie wird üblicherweise zur Herstellung eines elastischen Laminates verwendet. Ein solches Laminat ist besonders für Einwegartikel aus dem Hygienebereich wie beispielsweise Einwegwindeln, Einwegpants oder Einweginkontinenzprodukte für Erwachsene geeignet. Bei diesen Produkten besteht einerseits das Bedürfnis eines einfachen, kostengünstigen und ressourcenschonenden Aufbaus, wobei andererseits eine gute und zuverlässige Passform notwendig ist. Mit dem elastischen Laminat können dafür elastische Bereiche, beispielsweise Windelohren, Taillenbänder oder dergleichen gebildet werden. Auch für bestimmte medizinische Anwendungen wie Bandagen oder Wärmegürtel kommt ein Einsatz in Betracht. Wenn nachfolgend im besonderen Maße auf Windeln Bezug genommen wird, so ist diese Anwendung lediglich als exemplarisch zu betrachten.

Windeln, insbesondere für Babys und Kleinkinder, werden als Einwegmassenartikel produziert, sodass sich besonders hohe Anforderungen an eine effiziente Materialausnutzung sowie geringe Entstehungskosten ergeben.

Darüber hinaus ist jedoch gleichzeitig auch ein großes Maß an Funktionalität notwendig, damit unter allen Einsatzbedingungen ein hohes Maß an Sicherheit sowie ein zuverlässiger Schutz der windeltragenden Person sichergestellt werden kann. So müssen über Nacht große Mengen an Exkrementen aufgenommen und sicher zurückgehalten werden, wobei zur Abdichtung an der Taille sowie den Beinen eines Benutzers Einwegwindeln mit elastischen Elementen und Abschnitten vorgesehen sind.

Um elastische Seitenteile und insbesondere seitliche Windelverschlüsse zu bilden, sind elastische Laminat bekannt, welche an ihren gegenüberliegenden Außenseiten Lagen aus Nonwoven und dazwischen eine elastische Folie als Kern aufweisen. Dieser elastische Kern kann gemäß dem Stand der Technik beispielsweise durch eine vollflächig eingebundene elastische Folie oder auch durch elastische Stränge gebildet werden.

Diese elastische Folie ist entsprechend maßgeblich für das elastische Verhalten des Laminats, wobei die elastische Folie in bekannter Art und Weise als coextrudierter Mehrschichtverbund ausgebildet ist. Somit weist die elastische Folie zumindest eine Deckschicht aus einem nicht-elastischen Material auf, wobei die Elastizität der Folie über die daran anschließende elastische Folienschicht bewirkt wird. Mithilfe der nicht-elastischen Deckschicht wird gewährleistet, dass das coextrudierte Material während der Herstellung nicht miteinander verblockt, wodurch die Handhabung der elastischen Folie wesentlich vereinfacht wird.

Darüber hinaus dient die nicht-elastische Deckschicht auch der Aufrechterhaltung einer Bahnspannung. Hierbei ist zu beachten, dass die Folien üblicherweise entlang einer Maschinenrichtung (MD) während des Herstellungs- und Verarbeitungsprozesses transportiert werden, wobei zur Förderung der elastischen Folie in Maschinenrichtung eine Bahnspannung aufgebracht werden muss. Sofern die elastische Folie ausschließlich über die elastische Folienschicht gebildet würde, hätte diese Bahnspannung zufolge, dass sich das Material zumindest bereichsweise Längen und den Verarbeitungsprozess erschweren würde. Um die Elastizität der elastischen Folienschicht dennoch nutzen zu können, muss die gesamte Folie vorzugsweise in Maschinenrichtung gereckt werden. Hierdurch verdünnen sich die Deckschichten und werden beim Zurückstellen in Falten gelegt. Je nach Sprödheit bzw. Ausziehbarkeit der Deckschichten können diese auch alternativ oder ergänzend aufreißen. Durch das Recken der Folie wird folglich das elastische Verhalten der elastischen Folienschicht freigelegt. Ein solcher Schritt wird üblicherweise auch als Aktivieren bezeichnet, da durch die Reckung das elastische Verhalten der elastischen Folie aktiviert wird.

Das elastische Verhalten wird maßgeblich durch die verwendeten elastomeren Materialien bestimmt, wobei es aus dem Stand der Technik bekannt ist, Polyolefinelastomere (POE) zu verwenden. Derartige Elastomere weisen den Vorteil auf, dass sie während eines Herstellungsprozesses eines Laminats eine besonders gute Anbindung an die Außenlagen, insbesondere aus einem Nonwoven, ermöglichen. So erfolgt diese Anbindung heutzutage in der Praxis häufig durch Ultraschallschweißverfahren, bei der die Nonwovenlagen über oder durch die elastische Folie durch Aufbringen von Ultraschallenergie miteinander verschweißt werden. Polyolefinelastomere weisen hierbei im gedehnten Zustand eine sehr gute intrinsische Festigkeit auf, wodurch die elastischen Folien besonders widerstandsfähig sind z. B. gegenüber dem Einreißen beim Ultraschallbonden im gedehnten Zustand.

Alternativ haben sich in der Praxis auch elastische Folienschichten aus einem Styrolblockcopolymer (SBC) als besonders geeignet herausgestellt, wobei elastische Folienschichten auf Basis eines Styrolblockcopolymers bessere elastische Eigenschaften aufweisen als elastische Folienschichten auf Basis eines Polyolefinelastomers. Zugleich sinkt aber auch die intrinsische Festigkeit. Vor diesem Hintergrund haben sich in der Praxis elastische Folien als besonders vorteilhaft erwiesen, bei denen die elastische Folienschicht aus einer Polymermischung mit einer ersten Polymerkomponente A und einer zweiten Polymerkomponente B gebildet ist und wobei die erste Polymerkomponente ein Polyolefin, insbesondere ein elastomeres Polyolefin, oder eine Polyolefinmischung ist.

Die EP 3 126 140 B1 schlägt beispielsweise eine mehrschichtige elastische Folie vor, bei der eine elastomere Folienschicht zwischen zwei nicht-elastischen Deckschichten angeordnet ist. Die elastische Folienschicht besteht hierbei im Wesentlichen aus einem elastomeren Polyolefin, wobei auch bis zu 30 % einer zweiten Polymerkomponente B vorgesehen sein kann, wobei es sich vornehmlich um ein Styrol-Ethylen-Butylen-Styrol (SEBS) handelt. Darüber hinaus kann auch eine gewisse Menge inorganischer Partikel z. B. Ton vorgesehen sein. Hierdurch soll die Festigkeit der elastischen Folienschicht verbessert werden. Insbesondere soll sich hierdurch eine höhere Bruchfestigkeit bei einer geringeren Bruchdehnung ergeben.

Auch aus der EP 4 032 703 A1 ist ein Mehrschichtverbund aus nicht-elastischen Außenschichten und einer elastischen Kernschicht bekannt, wobei die elastische Kernschicht ein Polyolefin sowie ein Styrolblockcopolymer enthält. Darüber hinaus ist auch ein Anteil von bis zu 20 % eines Weichmachers vorgesehen. Hierbei handelt es sich insbesondere um Öl. Derartige Weichmacher bewirken, dass die Folie weicher und flexibler ist.

Wenn gleich sich die bislang bekannten elastischen Folien grundsätzlich bewährt haben, so führt der Kompromiss aus gutem elastischen Verhalten einerseits und guter Verarbeitbarkeit andererseits dazu, dass die daraus gebildeten Folien sowie die herausgebildeten elastischen Laminate ein elastisches Verhalten aufweisen, welches gerade bei dynamischen Belastungen von Nachteil ist.

Vor diesem Hintergrund liegt der Erfindung die Aufgabe zugrunde, eine elastische Folie anzugeben, welche sich durch ein verbessertes elastisches Verhalten sowie durch eine kostengünstige und ressourcenschonende Ausgestaltung auszeichnet. Zugleich soll auch das Prozessverhalten der Folie während der Herstellung verbessert werden.

Gegenstand und Lösung dieser Aufgabe ist eine elastische Folie gemäß Patentanspruch 1. Demnach ist vorgesehen, dass neben der ersten Polymerkomponente A, bei der sich um ein Polyolefin insbesondere um eine Polyolefinelastomer handelt, eine zweite Polymerkomponente B vorgesehen ist. Bei dieser zweiten Polymerkomponente B handelt es sich um ein Styrolblockcopolymer, welches in einer Menge zwischen 28 Gew.-% und 60 Gew.-% in der Polymermischung vorliegt und wobei die Polymermischung weniger als 1 Gew.-% inorganische und weniger als 5 Gew.-% organische Restbestandteile enthält.

Hierbei geht die Erfindung von der Erkenntnis aus, dass durch einen vergleichsweise hohen Anteil von Styrolblockcopolymeren und einer gleichzeitigen Reduzierung von Restbestandteilen ein besonders gutes elastisches Verhalten erzielen lässt. Entsprechend kann durch Erhöhung der zweiten Polymerkomponente im Wesentlichen sowohl auf organische und inorganische Restbestandteile verzichtet werden, sodass die zweite Polymermischung im Wesentlichen ausschließlich aus einem Polyolefin und aus einem Styrolblockcopolymer gebildet ist. Hierdurch kann eine elastische Folie gebildet werden, werden welche sich durch eine besonders geringe Rissneigung auszeichnet. Diese Eigenschaft ist gerade für die Weiterverarbeitung der Folie z. B. zu einem Laminat von großer Bedeutung. Insbesondere bei einer Ultraschallverschweißung wird die Folie punktuell aufgeschmolzen, ausgedünnt oder durchbrochen, wobei die Gefahr besteht, dass ausgehend von den Verbindungspunkten Risse in der elastischen Folie entstehen, so dass entsprechend eine niedrige Rissneigung der Folie von Vorteil ist. Darüber hinaus kann die Folie auch vor der Weiterverarbeitung deutlich stärker vorgedehnt bzw. aktiviert werden.

Eine alternative oder ergänzende Lösung sieht ferner vor, dass ausgehend von einer elastischen Folienschicht und zumindest einer ersten, mit der elastischen Folienschicht coextrudierten nicht-elastischen Deckschicht, ein elastischer Rückstellwert (ERV) nach einer Aktivierung um 300 % insbesondere in Maschinenrichtung, mehr als 0,4 beträgt.

Dieser elastische Rückstellwert lässt sich anhand einer Hysteresemessung an der Folie bestimmen. Üblicherweise erfolgt die Messung anhand einer sogenannten Drei-Zyklen-Hysterese, wobei eine Probe der elastischen Folie mit einer Probenbreite von 25,4 mm und einer Einspannlänge von 25,4 mm in einem ersten Überdehnungszyklus bis 300 % vorgedehnt wird. Diese erstmalige Überdehnung wird im Rahmen der Erfindung auch als Aktivierung bezeichnet, da hierdurch die nicht-elastischen Deckschichten ausgedünnt bzw. in Falten gelegt werden, sodass das elastische Verhalten der elastischen Folienschicht freigelegt wird. Grundsätzlich kann unmittelbar nach diesem ersten Zyklus die Folie einen elastischen Rückstellwert von zumindest 0,4 aufweisen. Bevorzugt werden aber im Anschluss zwei weitere Zyklen durchgeführt werden und erst nach dem dritten Zyklus anhand der Hysterese der elastische Rückstellwert ermittelt. Für diesen zweiten und dritten Zyklus wird die nunmehr vorgedehnte Probe der elastischen Folie neu eingespannt, wobei berücksichtigt wird, dass im Rahmen des ersten Zyklus eine plastische Verformung und damit Längung der Probe erfolgt ist. Nach Umspannen der Probe wird diese im zweiten und im dritten Zyklus um 120% gedehnt, wobei jeweils zwischen den einzelnen Zyklen die Folie für 60 Sekunden im entspannten Zustand gehalten wird. Im gedehnten Zustand wird die Folie hingegen für 30 Sekunden gehalten. Die Prüfgeschwindigkeit beträgt 500 mm/Minute. Nach jedem Zyklus lässt sich eine Hysteresekurve ermitteln, bei der die für die Dehnung erforderliche Kraft über die Dehnung aufgetragen wird, wobei die Dehnung üblicherweise in Prozent angegeben wird und sich auf die ursprüngliche Länge der Probe bezieht. Hierbei entspricht eine Dehnung um 100 % einer Verdoppelung der ursprünglichen Länge der Probe. Bei einer Dehnung von 0 % kehrt die Probe wieder in ihre ursprüngliche Länge zurück, sodass ein ausschließlich ein elastisches Verhalten beobachtet werden kann.

Anhand einer solchen Hysteresekurve lässt sich feststellen, dass die für die Dehnung erforderlichen Kräfte größer sind als die freiwerdenden Kräfte im Zuge der Entspannung. Entsprechend liegt daher eine sogenannte Ladungskurve oberhalb einer Entspannungskurve. Dieses Verhalten wird auch als viskoelastisches Verhalten bezeichnet, da ein Teil der für die Dehnung erforderlichen Energie durch innere Prozesse innerhalb der Folie dissipiert und nicht für die Entspannung genutzt werden kann. Diese Unterschiede zwischen Ladungskurve und Entspannungskurve werden auch als Verlustenergie bezeichnet, welche über den sogenannten ERV-Wert (elastic recovery value) bzw. durch den elastischen Rückstellwert beschrieben werden kann. Hierbei beschreibt der elastische Rückstellwert das Verhältnis zwischen der Fläche unterhalb der Entspannungskurve zu der Fläche unterhalb der Ladungskurve, sodass entsprechend ein ERV-Wert von max. 1 erreicht werden kann, wobei ein solcher Wert lediglich theoretisch erzielbar ist und ein ideales elastisches Verhalten beschreibt.

Im Rahmen der Erfindung ist es nunmehr möglich, einen elastischen Rückstellwert von zumindest 0,4, insbesondere von zunächst 0,45, zu erreichen, sodass entsprechend zumindest 40 % der für die Dehnung eingebrachten Energie auch während der Entspannung genutzt werden kann. Dies hat zur Folge, dass beispielsweise im Falle von Windeln bei dynamischen Belastungen das Material einerseits möglichst schnell in seinen Ursprungszustand zurückgeführt werden kann, sodass beispielsweise Bewegungen bei Babys schneller ausgeglichen werden können. Zugleich wird sichergestellt, dass das Material auch bei Bewegungen möglichst schnell eine ausreichende Kraft auf die anliegenden Körperteile aufbringt, sodass eine besonders gute Abdichtung gewährleistet werden kann.

Wenngleich die Menge der zweiten Polymerkomponente B zwischen 28 und 60 Gew.-% ausreichend sein kann, so liegt gemäß einer bevorzugten Ausgestaltung die zweite Polymerkomponente B in einer Menge zwischen 30 und 55 Gew.-%, besonders bevorzugt zwischen 32 und 50 Gew.-%, vor.

Die Erfindung zeichnet sich insbesondere dadurch aus, dass eine Polymermischung mit einer ersten und zweiten Polymerkomponente vorgesehen ist, wobei die zweite Polymerkomponente ein Styrol-Block-Copolymer ist. Bei diesem Styrol-Block-Copolymer kann es sich um ein Styrol-Isopren-Styrol-Blockcopolymer (SIS), ein Styrol-Butadien-Styrol-Blockcopolymer (SBS), ein Styrol-Ethylen-Butylen-Styrol (SEBS) oder um ein Styrol-Ethylen/Propylen-Styrol-Blockcopolymer (SEPS) handeln. Besonders bevorzugt ist als Styrol-Block-Copolymer ein Styrol-Ethylen-Propylen-Styrol-Blockcopolymer (SEEPS). Hierbei weist SEEPS die Besonderheit auf, dass es sich besonders gut mit der ersten Polymerkomponente A, bei der es sich insbesondere um ein Polyolefin oder eine Polyolefinmischung handelt, mischen lässt, sodass weitere Hilfsstoffe zur Herstellung einer Kompatibilität nicht erforderlich sind. Vor diesem Hintergrund können die beiden Primärkomponenten unmittelbar in der Extrusion gemischt werden, wodurch der Einsatz von Restbestandteilen, insbesondere von organischen Restbestandteilen signifikant gesenkt werden kann. SEEPS wird beispielsweise unter dem Handelsnamen Septon von Kuraray vertrieben.

Eine Weiterbildung der Erfindung sieht vor, dass die erste Polymerkomponente A ein Polyolefinelastomer (POE) enthält oder aus einem Polyolefinelastomer gebildet ist. Diese Elastomere zählen auch wie die Styrol-Blockcopolymere zu den thermoplastischen Elastomeren, welche sich einerseits durch Wärmezufuhr plastisch verformen lassen, wodurch beispielsweise die Herstellung im Zuge der Extrusion ermöglicht wird. Zugleich enthalten sie stets zwei Phasen, von denen die harte Phase für das Aufschmelzen und die weiche Phase für den elastomerartigen Charakter verantwortlich ist. Entsprechende Polyolefinelastomere werden beispielsweise unter dem Handelsnamen Vistamaxx von Exxon oder auch unter den Handelsnamen Infuse von Dow Chemical vertrieben.

Eine Weiterbildung der Erfindung sieht ferner vor, dass die erste Polymerkomponente A eine Polyolefinmischung aus einem ersten und einem zweiten Polyolefin ist, wobei das Verhältnis zwischen dem Gewichtsanteil des ersten zum Gewichtsanteil des zweiten Polyolefin zwischen 1:3 und 2:3 beträgt. Insbesondere besteht die Polyolefinmischung ausschließlich aus dem ersten und dem zweiten Polyolefin. Drüber hinaus sind zumindest das erste und/oder das zweite Polyolefin als Polyolefinelastomere ausgebildet.

Eine Weiterbildung der Erfindung sieht ferner vor, dass eine zweite nicht-elastische Deckschicht mit der elastischen Folienschicht koextrudiert ist, wobei die elastische Folienschicht zwischen der ersten und der zweiten Deckschicht angeordnet ist. Entsprechend bildet eine Art Sandwichverbund aus, bei dem die elastische Folienschicht als eine Kernschicht von den beiden nicht-elastischen Deckschichten eingeschlossen ist. Die beiden nicht-elastischen Deckschichten können hierbei grundsätzlich auf verschiedene Art und Weise ausgebildet sein, wobei jedoch bevorzugt vorgesehen ist, dass die beiden Deckschichten eine im Wesentlichen identische Ausbildung aufweisen. Insbesondere können die beiden Deckschichten eine identische Dicke und/oder eine übereinstimmende Materialzusammensetzung aufweisen.

Eine besonders bevorzugte Ausgestaltung sieht in diesem Zusammenhang vor, dass das Verhältnis der Dicke der elastischen Folienschicht jeweils zu der Dicke der ersten und/oder der zweiten Deckschicht zwischen 6:1 und 15:1, insbesondere zwischen 7:1 und 12:1, beträgt. Entsprechend zeichnet sich die Erfindung durch sehr dünne Deckschichten aus. Jeweils zu beachten ist, dass das elastische Verhalten der Folie im Wesentlichen aus dem Verhältnis der elastischen Folienschicht zu den nicht-elastischen Deckschichten bestimmt wird. Mit steigendem Verhältnis erhöht sich somit auch die elastische Leistung der daraus gebildeten elastischen Folie insgesamt. Entsprechend führen relativ dünne nicht-elastische Deckschichten zu einem besonders guten elastischen Verhalten der Folie.

Insbesondere ist vorgesehen, dass die erste und/oder die zweite Deckschicht eine Dicke zwischen 1 µm und 5 µm, bevorzugt zwischen 1,5 µm und 4 µm, besonders bevorzugt zwischen 2 µm und 3,5 µm aufweist. Im Gegensatz dazu weist die elastische Folienschicht insbesondere eine Dicke zwischen 15 und 43 µm, bevorzugt zwischen 17 und 30 µm, besonders bevorzugt zwischen 20 und 26 µm auf. Die Gesamtdicke der elastischen Folie beträgt bevorzugt zwischen 17 und 45 µm, besonders bevorzugt zwischen 20 und 36 µm, ganz bevorzugt zwischen 24 und 33 µm auf.

Die erste und/oder die zweite Deckschicht sind gemäß einer bevorzugten Ausgestaltung aus einem Polyolefin als Hauptbestandteil gebildet. Hierbei ist mit einem Hauptbestandteil gemeint, dass Polyolefin zumindest zu einem Anteil von 30 Gew.-% in der entsprechenden ersten oder zweiten Deckschicht vorliegt. Besonders bevorzugt liegt Polyolefin zumindest zu 45 Gew.-%, besonders bevorzugt zumindest zu 60 Gew.-% in der jeweiligen ersten und/oder zweiten Deckschicht vor. Besonders bevorzugt ist ein Bereich zwischen 45 und 70 Gew.-%.Bei den restlichen Bestandteilen kann es sich um organische oder auch inorganische Restbestandteile handeln. Insbesondere handelt es sich bei den restlichen Bestandteilen um Additive wie Stabilisatoren, Verarbeitungsmittel, Gleitmittel und/oder Antiblockmittel. Beispielsweise können die restlichen Bestandteile Talkum aufweisen oder aus Talkum gebildet sein. Der Anteil von Restbestandteilen kann zwischen 6 und 18 Gew.-% bezogen auf die Zusammensetzung der jeweiligen Deckschicht betragen. Bevorzugt beträgt der Anteil von Talkum 3 bis 15 Gew.-%, besonders bevorzugt zwischen 5 und 10 Gew.-%, bezogen auf die Zusammensetzung der jeweiligen Deckschicht.

Gemäß einer besonders bevorzugten Ausgestaltung der Erfindung sind die Polyolefine der ersten und/oder der zweiten Deckschicht Polyethylen oder Polypropylen. Im Falle von Polyethylen handelt es sich insbesondere um ein Polyethylen niedriger Dichte (LDPE) oder um ein lineares Polyethylen niedriger Dichte (LLDPE). Insbesondere LLDPE hat sich als besonders vorteilhaft erwiesen. Bei dem Polypropylen kann es sich beispielsweise um ein Homopolymer oder um ein Copolymer handeln. Bevorzugt sind in diesem Zusammenhang auch Mischungen aus Polyethylen, insbesondere linearem Polyethylen niedriger Dichte, mit einem Polypropylen, wobei der Anteil des Polypropylens zwischen 30 und 60 Gew.-% und/oder der Anteil des Polyethylens zwischen 30 und 60 Gew.-% bezogen auf die Polyolefinbestandteile betragen kann. Der Anteil von Restbestandteilen, insbesondere von Talkum, kann zwischen 15 und 25% bezogen auf den Anteil des Polypropylens betragen. Darüber hinaus kann die Folie vorteilhafterweise auch insgesamt relativ dünn ausgebildet werden, so dass im Sinne der Nachhaltigkeit Material gegenüber vergleichbaren Lösungen eingespart werden kann.

Wie zuvor bereits erläutert, sieht eine erfinderische Ausbildung der Erfindung vor, dass ein elastischer Rückstellwert (ERV) nach einer Aktivierung von 300 % mehr als 0,4, insbesondere mehr als 0,45, beträgt. Hierbei erfolgt die Aktivierung insbesondere durch Überdehnung der nicht-elastischen Deckschichten in Maschinenrichtung (MD), wobei der elastische Rückstellwert insbesondere dadurch erreicht werden kann, indem eine Polymermischung in der elastischen Folienschicht aus einem Polyolefinelastomer und einem Styrol-Blockcopolymer, insbesondere SEEPS, verwendet wird. Durch den hohen elastischen Rückstellwert kann die elastische Folie besonders schnell und mit vergleichsweise hoher Kraft nach einer Dehnung in ihren ursprünglichen Zustand zurückkehren, wodurch sich insbesondere ein hohes Maß an Passkomfort ergibt.

Neben der Bewertung des elastischen Rückstellwertes ist aber auch der sogenannte Permanent-Set von Bedeutung, welcher Auskunft darüber gibt, wie sich die elastische Folie nach einer erstmaligen Aktivierung dauerhaft verlängert. Hierzu wird der Permanentset üblicherweise anhand zweier nacheinander folgender Zyklen bestimmt, bei der die elastische Folie jeweils um 300 % ohne Umspannen gedehnt wird. In einem ersten Zyklus erfolgt die Dehnung bis 300 % mit einer Prüfgeschwindigkeit von 500 mm/min. Sodann wird die Folie für 30 Sekunden im gedehnten Zustand gehalten, anschließend entlastet und im entspannten Zustand noch mal für 60 Sekunden gehalten. Im Anschluss erfolgt eine erneute Dehnung um 300 % mit einer Prüfgeschwindigkeit von 500 mm/min. Die für die Messung verwendete Folie entspricht hinsichtlich ihrer Maße der Folie, welche auch für die HystereseMessung verwendet wurde. Entsprechend weist diese eine Probenbreite und Einspannlänge von 25,4 mm auf. Die Einspannlänge wird sodann mit der Probe nach der entsprechenden Dehnung verglichen, wobei die Längendifferenz bezogen auf die ursprüngliche Einspannlänge den Permanent-Set definiert. Sofern die Probe nach der entsprechenden Dehnung beispielsweise eine Länge von 50,8 mm aufweist, entspricht dies einem Permanent-Set von 100 %.

Ausgehend von einer solchen Messung beträgt der Permanent-Set nach der Aktivierung um 300 %, insbesondere nach einer zweimaligen Aktivierung um 300 %, bevorzugt weniger als 40 %, besonders bevorzugt weniger als 35 %, ganz besonders bevorzugt weniger als 30 %. Entsprechend wird somit eine elastische Folie bereitgestellt, welche einerseits einen hohen elastischen Rückstellwert und zugleich einen niedrigen Permanent-Set aufweist. Entsprechend können gegenüber bekannten Folien bei gleichem Permanent-Set höhere Rückstellkräfte erzeugt werden bzw. ist ein geringerer Permanent-Set erforderlich, um einen erforderlichen Rückstellwert zu erreichen.

In diesem Zusammenhang sieht eine Lösung mit eigenständiger erfindungsgemäßer Bedeutung vor, dass insbesondere bei einer gattungsgemäßen Folie, die zweite Polymerkomponente B ein Styrol-Ethylen-Ethylen-Propylen-Styrol-Blockcopolymer (SEEPS) ist und der Permanent-Set nach der Aktivierung um 300 % weniger als 40 %, besonders bevorzugt weniger als 35 %, ganz besonders bevorzugt weniger als 30 %, beträgt.

Es kann somit eine elastische Folie bereitgestellt werden, welche mit besonders dünnen Deckschichten hergestellt werden kann. Hierbei ist zu beachten, dass üblicherweise die Dicke der Schichten extrusionstechnisch limitiert ist und somit nicht beliebig dünne Deckschichten hergestellt werden können. Die Erfindung sieht allerdings nunmehr vor, dass die elastische Folie nicht nur in Maschinenrichtung (MD) sondern auch in Querrichtung (CD) aktiviert werden kann. Diese Aktivierung in Querrichtung dient allerdings nicht in erster Linie dazu, eine in Querrichtung dehnbare Folie herauszubilden. Vielmehr wird durch diese Aktivierung das Material der Deckschichten ausgedünnt, während der niedrige Permanent-Set gewährleistet, dass diese Ausdünnung im Wesentlichen keinen Einfluss auf die elastische Folienschicht hat. Hierdurch wird das Verhältnis zwischen der Dicke der einzelnen Schichten weiter zugunsten der elastischen Folienschicht verschoben, wodurch die elastische Leistung der Folie weiter verbessert wird. So ist es sogar möglich, Deckschichten zu erzeugen, die zumindest bereichsweise eine Dicke von weniger als 1 µm aufweisen.

Der bereits zuvor erwähnte elastische Rückstellwert kann insbesondere dadurch erreicht werden, indem durch eine geeignete Materialauswahl und Zusammensetzung ausgehend von einer Hysteresemessung die Differenz zwischen der Fläche unterhalb der Ladekurve und der Fläche unterhalb der Entspannungskurve nach der Aktivierung um 300 % bei einer Dehnung um 120 % minimiert wird. Insbesondere beträgt die Differenz weniger als 90 N/inch% besonders bevorzugt weniger als 80 N/inch%, ganz besonders bevorzugt weniger als 70 N/inch%.

Gemäß einer bevorzugten Ausgestaltung beträgt der Reibungskoeffizient der Folie gemäß ISO 8295 zwischen 0,2 und 0,6.

Gegenstand der Erfindung ist ferner ein elastisches Laminat mit zumindest einer Decklage aus einem Nonwoven und einer mit der Decklage verbundenen erfindungsgemäßen elastischen Folie. Üblicherweise werden im Zuge der Herstellung die Decklage sowie die elastische Folie über separate Nonwovenbahnen und Folienbahnen bereitgestellt und zur Ausbildung eines entsprechenden Laminates miteinander verbunden. Insbesondere ist vorgesehen, dass die elastische Folie zwischen zwei Decklagen aus einem Nonwoven angeordnet und mit diesen verbunden ist. Entsprechend bildet die elastische Folie den elastischen Kern des elastischen Laminats. Über die Decklagen aus Nonwoven wird eine weiche, angenehme und textilartige Oberfläche gebildet, wobei das Laminat zugleich kostengünstig hergestellt werden kann. Über die elastische Folie werden die elastischen Rückstelleigenschaften für einen sicheren Sitz und eine gute Abdichtung eines daraus gebildeten Hygieneartikels gewährleistet.

Zur Herstellung eines solchen Laminats sind verschiedene Ansätze bekannt. Beispielsweise können eine vorproduzierte elastische Folie und zumindest eine vorproduziert Decklage aus Nonwoven weitgehend spannungsfrei unter Einsatz von Druck und Temperatur, Klebstoffen oder durch ein Ultraschallverschweißen miteinander verbunden werden, wobei vorzugsweise ein Muster aus verbundenen und unverbundenen Bereichen vorgesehen ist.

Häufig ist jedoch noch während des Herstellungsprozesses einer Aktivierung der elastischen Folie oder des daraus gebildeten Laminates vorgesehen, wozu die Folie über das Laminat in einer gewünschten Dehnrichtung gedehnt wird. Insbesondere ist vorgesehen, dass die elastische Folie vor dem Verbinden mit der zumindest einen Decklage zumindest in Maschinenrichtung (MD) gedehnt wird. Dies kann beispielsweise durch zwei hintereinander angeordnete Walzenanordnungen ermöglicht werden, welche mit ansteigender Geschwindigkeit betrieben werden. Entsprechend bildet sich zwischen diesen beiden Walzenanordnungen eine größere Bahnspannung aus, welche zu der gewünschten Aktivierung der Folie führt. Sodann kann die elastische Folie im gedehnten Zustand durch die bereits genannten Verbindungsarten mit der zumindest einen Decklage verbunden werden. Ein solches Verfahren wird gemeinhin auch als Strechbonding bezeichnet.

Gemäß einem weiteren bekannten Verfahren wird die elastische Folie zunächst, z. B. mittels Ringrollen, voraktiviert und in zwei Streifen geschnitten. Die beiden Streifen werden dann jeweils zu einer zugeordneten Dehnungseinheit mit schräg stehenden Scheiben (Spreader) geführt und an diese Dehnungseinheit übergeben, wenn die beiden zueinander schräg gestellten Scheiben den geringsten Abstand aufweisen. Durch die Schrägstellung der Scheiben erfolgt dann die Dehnung der elastischen Streifen in Querrichtung (CD), wobei die elastischen Folienstreifen an ihren Rändern mittels Vakuum gehalten und an dem Spreader geführt sind.

Eine erste Nonwovenbahn wird auf einer zentralen Walze geführt, bevor dann der erste elastische Streifen von dem Spreader übergeben und auch mittels Vakuum festgehalten wird. Nachfolgend wird dann der zweite elastische Streifen parallel versetzt in gleicher Weise aufgelegt und festgehalten. Nach der parallelen Anordnung der beiden elastischen Streifen auf der ersten Nonwovenbahn kann dann eine zweite Nonwovenbahn aufgelegt werden. Nachfolgend erfolgt dann ein Verschweißen mittels Ultraschall.

Alternativ ist es auch möglich, die elastische Folie in einem zwar aktivierten aber nicht-gedehnten Zustand mit der Decklage bzw. mit den Decklagen zu verbinden. Darüber hinaus kann sodann auch das Laminat anschließend aktiviert werden. Dies erfolgt beispielsweise quer zur Maschinenrichtung. Hierzu kann das Laminat beispielsweise einer Dehnung mit Ringrollen zugeführt werden. Durch den wellenförmigen oder zick-zack-förmigen Verlauf des Walzenspaltes von ineinandergreifenden Ringrollen wird das eingeführte Material in einer Querrichtung verlängert, wodurch die dehnbare, jedoch nicht-elastische Decklage aus Nonwoven überdehnt, bleibend verformt und gegebenenfalls teilweise zerstört wird.

Die beschriebenen Herstellungsprozesses sind hierbei lediglich exemplarisch aufgeführt, wobei selbstverständlich auch weitere Verbindungsformen und Herstellungsprozesse für Laminate aus einer elastischen Folie und einer Decklage möglich sind.

Darüber hinaus ist es auch nicht zwingend erforderlich, dass die elastische Folie vollflächig an der zumindest einen Decklage bzw. an den Decklagen anliegt. Gerade bei Ausgestaltungen mit zwei außenliegenden Decklagen ist es auch denkbar, dass die elastische Folie lediglich bereichsweise zwischen den Nonwovenbahnen in Abschnitten angeordnet ist. Bei diesen Abschnitten kann es sich beispielsweise um mehrere in Querrichtung zueinander beabstandete Stränge handeln, wobei zwischen den Strängen die Decklagen nicht über die elastische Folie sondern unmittelbar miteinander verbunden sind. Gemäß einer solchen Ausgestaltung ist also die Elastizität lediglich in Abschnitten des elastischen Laminates vorgesehen. Hierbei reicht es dann auch aus, wenn die Decklagen lediglich in den Bereichen aktiviert sind, in denen auch die elastische Folie angeordnet ist.

Gegenstand der Erfindung ist darüber hinaus ein Hygieneartikel mit einem erfindungsgemäßen elastischen Laminat. Bei diesem Hygieneartikel handelt es sich insbesondere um einen elastischen Bund, elastische Verschlusselemente oder sonstige elastische Elemente einer Windel.

Im Folgenden wird die Erfindung anhand eines exemplarischen Beispiels näher erläutert. Die Figuren zeigen:
- Fig. 1: einen exemplarischen Aufbau einer erfindungsgemäßen elastischen Folie
- Fig. 2A, 2B, 2C, 2D: Hysteresekurven für zwei exemplarische Folien sowie für zwei Vergleichsbeispiele aus dem Stand der Technik
- Fig. 3: ein schematisch dargestelltes Verfahren zur Herstellung eines erfindungsgemäßen elastischen Laminates.

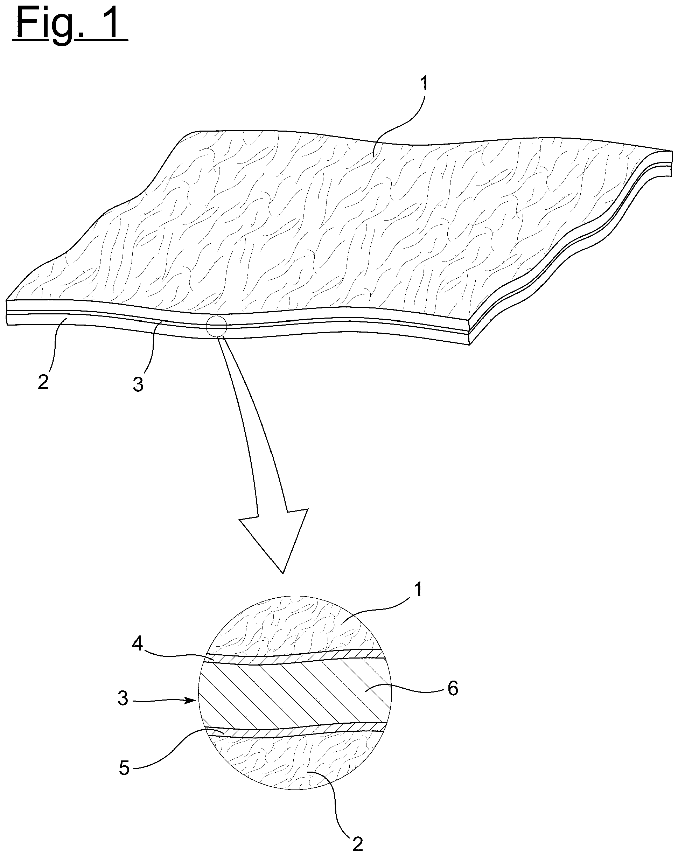

Die Fig. 1 zeigt ein erfindungsgemäßes elastisches Laminat mit einer oberen ersten Decklage 1 sowie einer zweiten unteren Decklage 2, welche aus einem Nonwoven gebildet sind und welche eine als Kernlage ausgebildete elastische Folie 3 einschließen. Ein solches elastisches Laminat kommt insbesondere bei Hygieneartikeln zum Einsatz, da die äußeren Decklagen 1, 2 eine angenehme weiche Oberfläche bilden, während über die innenliegende elastische Folie 3 ein Rückstellverhalten ausgebildet werden kann.

Die Decklagen 1, 2 können in verschiedener Art und Weise mit der elastischen Folie 3 verbunden sein, wobei insbesondere eine Verbindung mittels Klebstoff oder aber mittels Ultraschallschweißen in Betracht kommt. Im Falle eines Ultraschallschweißens erfolgt die Verbindung an mehreren nicht näher in der Fig. 1 dargestellten Verbindungspunkten, wobei an diesen Verbindungspunkten die Decklagen 1, 2 entweder unmittelbar durch die elastische Folie 3 hindurch oder über die elastische Folie 3 miteinander verbunden sind. Da die elastische Folie 3 üblicherweise unter Spannung mit den Decklagen 1, 2 verbunden wird, bilden sich im Zuge des Ultraschallschweißens an den Verbindungspunkten Löcher in der elastischen Folie 3, wodurch eine besonders gute Atmungsaktivität durch das Laminat hindurch erreicht werden kann. Allerdings stellen diese Löcher auch Schwachstellen innerhalb der Folie 3 dar, da sich ausgehend von diesen Löchern Risse in der Folie 3 bilden können. Die im Rahmen des erfindungsgemäßen elastischen Laminats verwendete elastische Folie 3 weist hierbei eine sehr geringe Rissneigung auf und kann unter besonders hohen Spannungen mit den Decklagen 1, 2 verbunden werden.

Bei der elastischen Folie 3 handelt es sich um eine mehrschichtig coextrudierte Folie, deren einzelnen Schichten insbesondere in der Detailansicht in einem Querschnitt deutlicher dargestellt sind. Demnach weist die elastische Folie 3 eine erste und eine zweite nicht-elastische Deckschicht 4, 5 auf, welche eine elastische Folienschicht 6 als Kernschicht einschließen. Das elastische Verhalten der elastischen Folie 3 sowie des gesamten elastischen Laminats wird folglich durch die elastische Folienschicht 6 bestimmt, wobei bereits anhand der Fig. 1 deutlich wird, dass die elastische Folie 3 vergleichsweise dünne, nicht-elastische Deckschichten 4, 5 vorsieht, sodass das elastische Verhalten der gesamten elastischen Folie 3 maßgeblich über die elastische Folienschicht 6 bestimmt wird.

Da das erfindungsgemäße Laminat vorzugsweise in Hygieneartikeln wie z. B. Windeln eingesetzt werden soll, ist es hierbei entscheidend, dass das Laminat und entsprechend auch die verwendete elastische Folie 3 ein gutes Rückstellverhalten aufweisen. Hierbei ist zu beachten, dass auch bei dynamischen Belastungen eines solchen Laminats stets gewährleistet werden muss, dass die daraus gebildeten Hygieneartikel dichtend und zugleich mit einem hohen Tragekomfort an den entsprechenden Körperstellen anliegen, um ein Auslaufen z. B. von Exkrementen zu vermeiden. Allerdings zeigt sich bei den aus der Praxis bekannten elastischen Folien 3 häufig ein stark ausgeprägtes viskoelastisches Verhalten. Dies bedeutet, dass die für die Dehnung erforderliche Energie häufig deutlich höher ist als die bei einer Entspannung freiwerdende Energie. Dieser Energieverlust führt zu einer Dämpfung der elastischen Eigenschaften, sodass beispielsweise bei einer Belastung eines entsprechenden Hygieneartikels das Material verformt wird, aber bei einer Entlastung nicht schnell genug und mit ausreichender Dichtkraft an den entsprechenden Körperstellen anliegt.

Die erfindungsgemäße elastische Folie 3 sieht nunmehr vor, dass die elastische Folie 3 einen elastischen Rückstellwert (ERV) aufweist, welcher nach einer Aktivierung um 300 % mehr als 0,4, bevorzugt mehr als 0,45, beträgt. Dieser elastische Rückstellwert vergleicht die freiwerdende Energie mit der zur Dehnung erforderlichen Energie bei einer Dehnung um 120 %, wobei ein elastischer Rückstellwert von 1 ein ideales Gummi darstellen würde, wobei aber in der Realität dissipativen Effekte zu einem Energieverlust führen, so dass dieser Wert selbstverständlich in der Praxis nicht erreichbar ist. Die erfindungsgemäße elastische Folie 3 kann nunmehr zumindest 40 % oder mehr von der zur Dehnung erforderlichen Energie für die Rückstellung nutzen, sodass eine wesentliche Verbesserung gegenüber bereits aus dem Stand der Technik bekannten Lösungen erreicht wird.

Um einen solchen hohen elastischen Rückstellwert zu erreichen, kann die elastische Folienschicht 6 aus einer Polymermischung mit einer ersten Polymerkomponente A und einer zweiten Polymerkomponente B gebildet sein, wobei die erste Polymerkomponente A ein Polyolefin, insbesondere ein Polyolefinelastomer, ist und wobei es sich bei der zweiten Polymerkomponente B um ein Styrol-Block-Copolymer handelt, wobei sich insbesondere Styrol-Ethylen-Ethylen-Propylen-Styrol-Blockcopolymere (SEEPS) als besonders vorteilhaft erwiesen haben, und wobei die Polymerkomponente B vorzugsweise in einer Menge zwischen 32 Gew.-% und 55 Gew.-% in der Polymermischung vorliegt. Darüber hinaus sieht die Erfindung auch vor, dass die elastische Folienschicht 6 vorzugsweise ausschließlich aus der ersten und der zweiten Polymerkomponente gebildet ist, sodass entsprechend keine oder nur unwesentliche Restbestandteile in der elastischen Folienschicht 6 vorliegen. Insbesondere ist vorgesehen, dass die inorganischen Restbestandteile weniger als 1 Gew.-% und die organischen Restbestandteile weniger als 5 Gew.-% innerhalb der Polymermischung ausmachen. Hier kommt gerade SEEPS als zweite Polymerkomponente B eine besondere Bedeutung zu, da es sehr gut mit einem Polyolefinelastomer mischbar ist, sodass weitere Verarbeitungsmittel nicht erforderlich sind. Durch die Verringerung der Restbestandteile wird eine Polymermischung bereitgestellt, welche ausschließlich aus elastischen Bestandteilen gebildet ist, sodass hierdurch die elastische Folienschicht 6 sowie die Folie 3 und das Laminat ein besonders gutes Rückstellverhalten aufweisen.

Die Tabellen 1 zeigt vier verschiedene elastische Folien 3, wobei die Folien 1 und 2 in bekannter Art und Weise ausschließlich aus einem Polyolefin-Elastomer in der elastischen Folienschicht 6 gebildet sind, während die Folie 3 in der elastischen Folienschicht 6 einen Anteil von 41 Gew.-% eines Styrol-Ethyen-Ethylen-Propylen-Styrol-Blockcopolymers (SEEPS) und zu 59 Gew.-% ein Polyolefinelastomers aufweist. Die Folie 4 weist in der elastischen Folienschicht 6 einen Anteil von 45 Gew.-% eines Styrol-Ethyen-Ethylen-Propylen-Styrol-Blockcopolymers (SEEPS) und zu 55 Gew.-% ein Polyolefinelastomers auf. Darüber hinaus wurde die Folie 4 in Querrichtung (CD) aktiviert, wodurch die nicht-elastischen Deckschichten 4, 5 ausgedünnt wurden, sodass die Dicke der Folie zu 82 % durch die elastische Folienschicht 6 definiert wird, während gemäß den Folien 1 bis 3 die elastische Folienschicht 6 lediglich 80 % der Dicke der Folie 3 ausmacht.

**Tabelle 1**

| Folie | Folienaufbau A/B/A [%] | Anteil SEEPS in B [%] | Anteil POE in B [%] | Dicke [µm] | CD-Aktiviert |
|---|---|---|---|---|---|
| 1 | 10/80/10 | 0 | 100 | 30 | nein |
| 2 | 10/80/10 | 0 | POE-A: 50, POE-B: 50 | 30 | nein |
| 3 | 10 / 80 / 10 | 41 | 59 | 30 | nein |
| 4 | 9/82/9 | 45 | 55 | 30 | ja |

Der genaue Aufbau der Folien 1 und 2 ist in den Tabellen 2 und 3 und der Aufbau der Folien 3 und 4 in den Tabellen 4 und 5 aufgeführt, wobei die Zusammensetzung der Deckschichten 4 bei den Folien 3 und 4 identisch ist, sodass sich im Wesentlichen Unterschiede in der elastischen Folienschicht 6 ergeben. Darüber hinaus sind gemäß der Folie 4 auch die Deckschichten 4, 5 geringfügig dünner als in den Folien 1 bis 3.

**Tabelle 2**

| Folie 1 | Dicke [µm] | Zusammensetzung |
|---|---|---|
| Deckschicht A (4) | 3,0 | 40 % LLDPE |
| | | 40 % PP |
| | | 8 % Talkum |
| | | 10 % LDPE |
| | | 2 % Additive |
| elastische Folienschicht B (6) | 24,0 | 100 % POE |
| Deckschicht A (5) | 3,0 | 40 % LLDPE |
| | | 40 % PP |
| | | 8 % Talkum |
| | | 10 % LDPE |
| | | 2 % Additive |

**Tabelle 3**

| Folie 2 | Dicke [µm] | Zusammensetzung |
|---|---|---|
| Deckschicht A (4) | 3,0 | 40 % LLDPE |
| | | 40 % PP |
| | | 8 % Talkum |
| | | 10 % LDPE |
| | | 2 % Additive |
| elastische Folienschicht B (6) | 24,0 | POE-A: 50, POE-B: 50 |
| Deckschicht A (5) | 3,0 | 40 % LLDPE |
| | | 40 % PP |
| | | 8 % Talkum |
| | | 10 % LDPE |
| | | 2 % Additive |

**Tabelle 4**

| Folie 3 | Dicke [µm] | Zusammensetzung |
|---|---|---|
| Deckschicht A (4) | 3,0 | 55 % LLDPE |
| | | 35 % PP |
| | | 8 % Talkum |
| | | 2 % Additive |
| elastische Folienschicht B (6) | 24,0 | 59% POE; 41 % SEEPS |
| Deckschicht A (5) | 3,0 | 55 % LLDPE |
| | | 35 % PP |
| | | 8 % Talkum |
| | | 2 % Additive |

**Tabelle 5**

| Folie 4 | Dicke [µm] | Zusammensetzung |
|---|---|---|
| Deckschicht A (4) | 2,8 | 55 % LLDPE |
| | | 35 % PP |
| | | 8 % Talkum |
| | | 2 % Additive |
| elastische Folienschicht B (6) | 24,4 | 55% POE; 45 % SEEPS |
| Deckschicht A (5) | 2,8 | 55 % LLDPE |
| | | 35 % PP |
| | | 8 % Talkum |
| | | 2 % Additive |

Anhand der Tabellen 2 bis 5 wird deutlich, dass die nicht-elastischen Deckschichten 4, 5 im Wesentlichen aus einem Polyolefin bestehen, wobei es sich gemäß den Tabellen 4 und 5 bei den Folien 3 und 4 um eine Polyolefinmischung aus 55 % linearen Polyethylen niedriger Dichte (LLDPE) und 35 Gew.-% Polypropylen handelt. Bei den Folien 1 und 2 kommt gemäß den Tabellen 2 und 3 ein etwas niedriger Anteil an linearen Polyethylen niedriger Dichte zum Einsatz. Dafür sehen die Außenschichten einen Anteil von 10 % an Polyethylen niedriger Dichte vor. Hierbei hat sich gezeigt, dass ein höherer Anteil an linearen Polyethylen niedriger Dichte (LLDPE) vorteilhaft im Hinblick auf die Ausziehbarkeit der dünnen Außenschichten ist. Es scheint auch vorteilhaft zu sein, dass das LLDPE der Folien 3 und 4 einen höheren molekularen Verzweigungsgrad aufweist, da im Extrusionsprozess gleichmäßig dünne Außenschichten ausgebildet werden konnten, die gut dehnbar sind, um die Folie nachträglich zu aktivieren.

Als lineares Polyethylen niedriger Dichte kam in den Beispielen ein Polymer von Exxon zum Einsatz. Dieses Material weist eine Dichte 0,936 g/cm³ und ein Schmelzindex (MFI) (190°C/2,16 kg) von 5,0 g/10N auf. Es handelt sich um ein Ethylen-Buten-Copolymer mit einem Schmelzpunkt von 125 °C und einem Biegemodul von 470 MPa auf.

Als Polypropylen wurde ein Polypropylen mit einer Dichte von 0,910 g/cm³ und einer Schmelzflussrate (MFR) (230°C/2,16 kg) von 26 g/10n verwendet. Das Polypropylen wies eine Schmelztemperatur von einer 161 °C auf.

Als Polyethylen niedriger Dichte (LDPE) wurde für die Folien 1 und 2 ein Ethylen-Octen-Copolymer mit einer Dichte von 0,919 g/cm³ und einem Schmelzindex (MFI) (230 °C/2,16 kg) von 6g/10n verwendet.

Neben den beiden Polyolefinkomponenten wurden darüber hinaus Talkum sowie Verarbeitungsadditive in der Polymermischung verwendet. Beide Deckschichten 4, 5 sind hierbei identisch ausgebildet.

Für die elastische Folienschicht 6 wurde als Polyolefinelastomer in allen vier Folien Vistamaxx 6102 verwendet. Das Material weist eine Dichte von 0,862 g/cm³ sowie eine Schmelzindex (MFI) (190 °C/2,16 kg) von 1,4 g/10n sowie einen Schmelzindex (MFI) (230 °C/2,16 kg) von 3,0 g/10b auf. Die Härte beträgt 67 gemäß Schore-A. Der Ethylenanteil beträgt 16 % und das Biegemodul 14 MPa.

Als Styrol-Ethylen-Ethylen-Propylen-Styrol-Blockcopolymer (SEEPS) wurde das Polymer Septon F4902 von Kuraray verwendet. Es handelt sich um ein hydriertes Triblock-Copolymer mit Styrol, Isopren und Butadien. Das Material weist eine Dichte von 0,9 g/cm³, einen Schmelzindex (MFI) (200 °C/ 5 kg) von 2,9 g/10n und eine Härte von 56 Schore-A auf.

Die Folien wurden alle mittels Cast-Coextrusion hergestellt. Selbstverständlich ist es im Rahmen der Erfindung aber auch möglich, die Folien mittels Blasfolienverfahren herzustellen.

Die vier Folien wurden nach der Herstellung sowohl einer Hysteresemessung gemäß der 3-Zyklen-Hysterese sowie einer Messung des Permanent-Sets unterzogen. Für die Hysteresemessung wurde eine Probenbreite von 25,4 mm und einer Einspannlänge von 25,4 mm bei einer Prüfgeschwindigkeit von 500 mm/min um 300 % gedehnt und für 30 Sekunden im gedehnten Zustand gehalten. Sodann wurde die Folie entspannt und für 60 Sekunden im entspannten Zustand gehalten. Nach einem Umspannen der Probe wurde diese bis 120 % gedehnt und erneuert für 30 Sekunden im gedehnten Zustand gehalten. Nach einem Entspannen und eine Haltezeit von weiteren 60 Sekunden erfolgte eine weitere Dehnung um 120 % sowie ein daran anschließendes Entspannen.

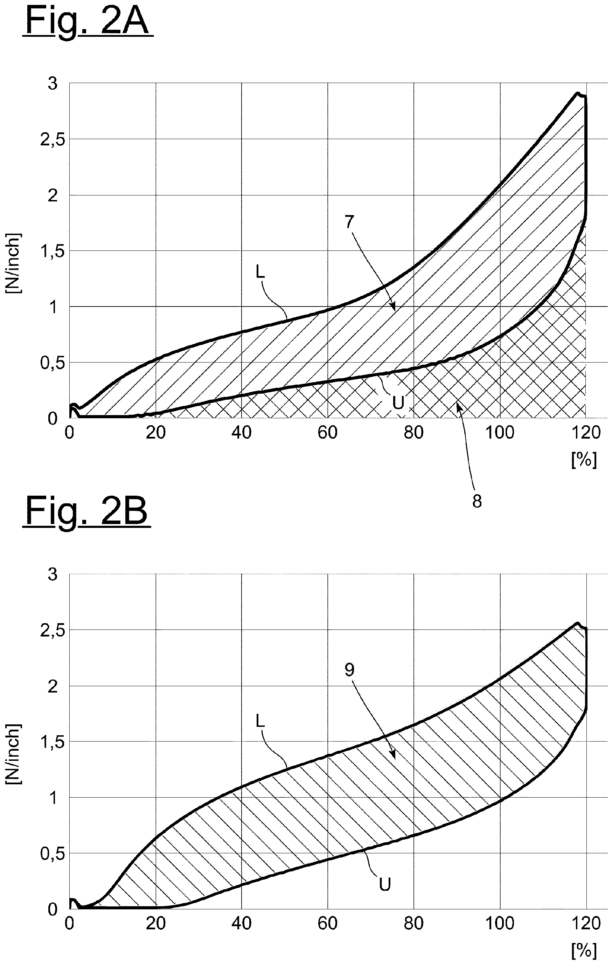

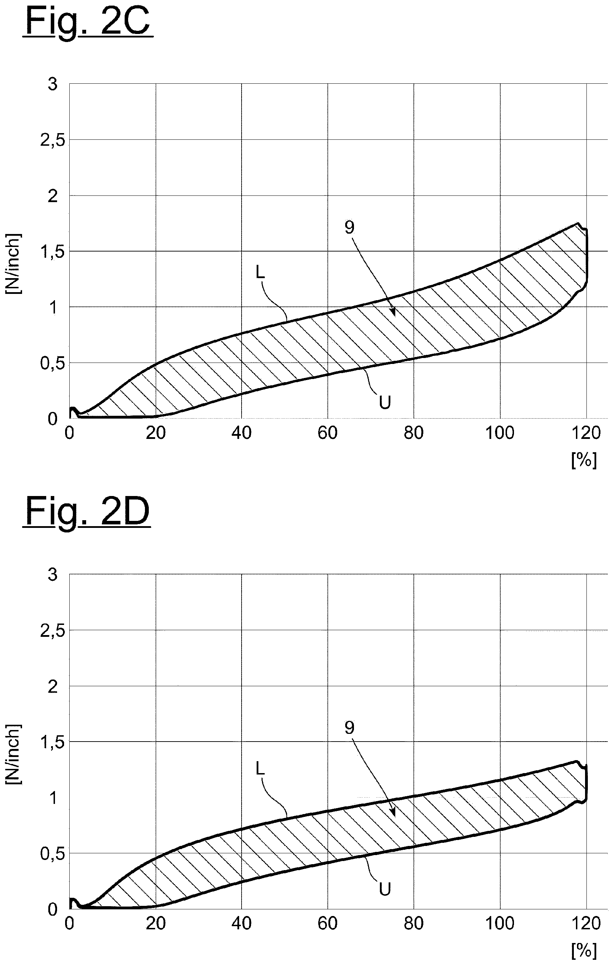

Die Figuren 2A, 2B, 2C, 2D zeigen den Kraftverlauf über die entsprechende Verlängerung der Probe für alle vier aufgeführten Folien, wobei die Figur 2A die Hysteresekurve für die Folie 1, die Fig. 2B die Hysteresekurve für die Folie 2, die Fig. 2C die Hysteresekurve für die Folie 3 und die Fig. 2D die Hysteresekurve für die Folie 4 zeigt. Sämtliche Hysteresekurven wurden während des dritten Zyklus während der Hysteresemessung aufgezeichnet.

Anhand der Fig. 2A, 2B, 2C, 2D wird deutlich, dass für sämtliche Messungen eine obere Ladekurve L sowie eine untere Entlastungskurve U vorgesehen ist, wobei die Ladekurve L die Kraft beschreibt, welche zur Dehnung der Probe um 120° erforderlich ist und wobei die Entlastungskurve U die Kraft aufzeigt, welche im Zuge der Entlastung der Probe frei wird. Entsprechend zeigen die Hysteresekurven, dass zwischen dem Spannen und Entspannen dissipative Effekte dazu führen, dass die zunächst eingebrachte Energie nicht vollständig während des Entlastens genutzt werden kann. Diese Verlustenergie kann insbesondere durch den sogenannten elastischen Rückstellwert (ERV) quantifiziert werden. Dies erfolgt exemplarisch anhand der Fig. 2A über einen Vergleich der Flächen unterhalb der Ladungskurve L und der Entlastungskurve U.

Die Flächen unterhalb der Entlastungskurve 7 und unterhalb der Entlastungskurve 8 sind in der Fig. 2A dargestellt, wobei dies selbstverständlich auch für alle weiteren Folien dargestellt werden kann. Die Fläche unterhalb der Entlastungskurve 8 wird sodann durch die Fläche unterhalb der Ladungskurve 7 geteilt, sodass theoretisch Maximalwerte von 1 erreichbar sind.

Die elastischen Rückstellwerte sind sodann in der Tabelle 6 aufgezeigt. Insgesamt wird deutlich, dass im Vergleich zu den aus dem Stand der Technik bekannten Folien 1 und 2 mithilfe der erfindungsgemäßen elastischen Folie 3 deutlich höhere elastische Rückstellwerte von über 0,4 erzielbar sind. Insbesondere die Folie 4 erzeugt einen elastischen Rückstellwert von 0,49, sodass entsprechend fast die Hälfte der Energie, welche für die Dehnung erforderlich ist, im Zuge der Entlastung wieder frei wird und genutzt werden kann.

**Tabelle 6**

| Folie | 1 | 2 | 3 | 4 |
|---|---|---|---|---|
| ERV | 0,34 | 0,37 | 0,43 | 0,49 |

Dieser positive Effekt wird auch bei einem visuellen Vergleich der Hysteresekurven deutlich, wobei insbesondere die Differenzfläche 9 zwischen der Ladungskurve L und der Entlastungskurve U bei den erfindungsgemäßen Folien 3 gemäß der Abbildung 2C, 2D deutlich kleiner ist als bei den Vergleichsbeispielen gemäß den Fig. 2A und 2B. Diese Unterschiede sind quantitativ in der Tabelle 7 aufgeführt, wobei deutlich wird, dass die Ladungskurven L und die Entlastungskurven U deutlich näher aneinander liegen, wobei gerade im Fall der Folie 4 Werte von 49,5 N/inch% möglich sind, während die Werte für die Vergleichsbeispiele im Wesentlichen bei annähernd 100 N/inch% liegen.

**Tabelle 7**

| Folie | 1 | 2 | 3 | 4 |
|---|---|---|---|---|
| Differenzfläche (9) [N/inch%] | 95,2 | 100,9 | 64,7 | 49,5 |

Neben dem elastischen Rückstellwert (ERV) der Permanent-Set durch die erfindungsgemäße Folie 3 deutlich verbessert werden, wobei die Werte für den Permanent-Set in der nachfolgenden Tabelle 8 aufgeführt sind. Hierbei liegt der Permanent-Set nach einer zweimaligen Dehnung um 300 % bei 22,6 % gemäß der Folie 4, wobei im Falle der Vergleichsbeispiele der Permanent-Set mehr als das Doppelte beträgt.

**Tabelle 8**

| Folie | 1 | 2 | 3 | 4 |
|---|---|---|---|---|
| Permanent-Set [%] | 47,6 | 41,7 | 30,8 | 22,8 |

Ferner zeigt die nachfolgende Tabelle 9 die mechanischen Eigenschaften der verschiedenen Folien, wobei deutlich wird, dass trotz der verbesserten elastischen Eigenschaften keine Verschlechterungen hinsichtlich der mechanischen Eigenschaften zu erwarten sind.

**Tabelle 9**

| Folie | Zugfestigkeit [N/inch] | Bruchdehnung MD [%] | MD-Dehnung bei 5N [%] | MD-Dehnung bei 10N [%] |
|---|---|---|---|---|
| 1 | 22 | 608 | 283 | 443 |
| 2 | 28 | 465 | 207 | 339 |
| 3 | 24 | 574 | 221 | 361 |
| 4 | 18 | 482 | 262 | 379 |

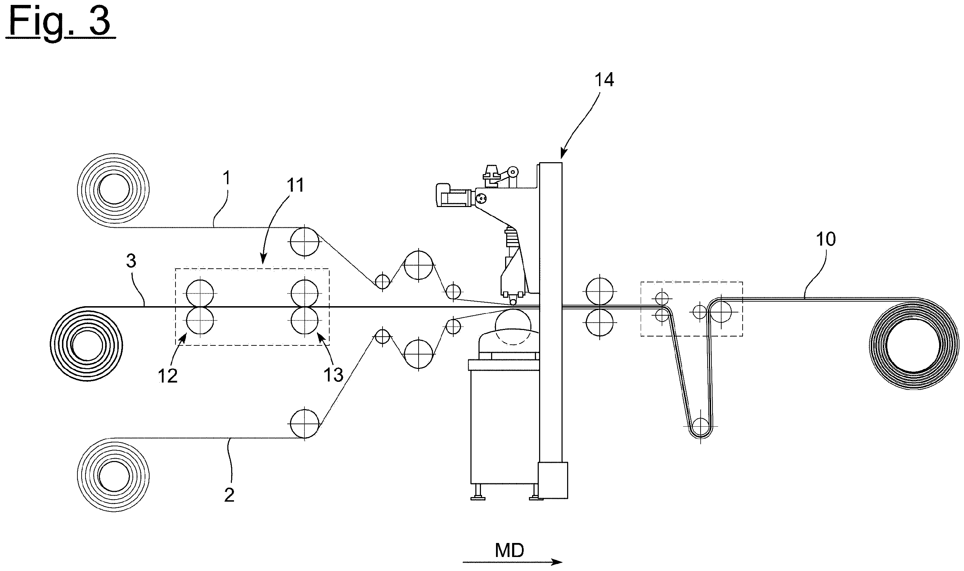

Die Figur 3 zeigt exemplarisch ein Herstellungsverfahren zur Herstellung einer Laminatbahn 10, welche einen gemäß der Fig. 1 dargestellten Aufbau aufweist. Hierzu werden einerseits die Decklagen 1, 2 sowie die elastische Folie 3 in Form einer Bahn entlang der Maschinenrichtung (MD) geführt, wobei die elastische Folie 3 vor der Verbindung mit den Decklagen 1, 2 in einer Aktivierungseinheit 11 in Maschinenrichtung (MD) aktiviert wird. Dies erfolgt über zwei mit unterschiedlicher Drehgeschwindigkeit rotierenden Walzenanordnungen 12, 13, wobei die Walzenanordnung 13 eine höhere Rotationsgeschwindigkeit aufweist als die Walzenanordnung 12, sodass entsprechend die elastische Folie 3 zwischen diesen Walzenanordnungen 12, 13 gestreckt wird. Sodann erfolgt die Verbindung im gedehnten Zustand mit den Decklagen 1, 2 in einer Ultraschallschweißanlage 13.

## Patentansprüche

**1.** Elastische Folie (3) mit einer elastischen Folienschicht (6) und zumindest einer ersten, mit der elastischen Folienschicht coextrudierten nicht-elastischen Deckschicht (4, 5), wobei die elastische Folienschicht (6) aus einer Polymermischung mit einer ersten Polymerkomponente A und einer zweite Polymerkomponente B gebildet ist und wobei die erste Polymerkomponente ein Polyolefin oder eine Polyolefinmischung ist,
**dadurch gekennzeichnet, dass**
die zweite Polymerkomponente B ein Styrol-Block-Copolymer ist, welches in einer Menge zwischen 28 Gew.-% und 60 Gew.-% in der Polymermischung vorliegt und wobei die Polymermischung weniger als 1 Gew.-% inorganische und weniger als 5 Gew.-% organische Restbestandteile enthält.

**2.** Elastische Folie (3) nach Anspruch 1, **dadurch gekennzeichnet, dass** die zweite Polymerkomponente in einer Menge zwischen 30 Gew.-% und 55 Gew.-% in der Polymermischung vorliegt.

**3.** Elastische Folie (3) nach Anspruch 1 oder 2, **dadurch gekennzeichnet, dass** die zweite Polymerkomponente B ein Styrol-Ethylen-Ethylen-Propylen-Styrol-Blockcopolymer (SEEPS) ist.

**4.** Elastische Folie (3) nach einem der Ansprüche 1 bis 3, **dadurch gekennzeichnet, dass** die erste Polymerkomponente A eine Polyolefinmischung aus einem ersten und einem zweiten Polyolefin ist, wobei das Verhältnis zwischen dem Gewichtsanteil des ersten zum Gewichtsanteil des zweiten Polyolefins zwischen 1:3 und 2:3 beträgt.

**5.** Elastische Folie (3) nach einem der Ansprüche 1 bis 4, **dadurch gekennzeichnet, dass** die erste Polymerkomponenten A ein Polyolefin-Elastomer enthält oder aus einem Polyolefin-Elastomer gebildet ist.

**6.** Elastische Folie (3) nach einem der Ansprüche 1 bis 5, **dadurch gekennzeichnet, dass** eine zweite nicht-elastische Deckschicht (4, 5) mit der elastischen Folienschicht (6) coextrudiert ist, wobei die elastische Folienschicht (6) zwischen der ersten und der zweiten Deckschicht (4, 5) angeordnet ist.

**7.** Elastische Folie (3) nach einem der Ansprüche 1 bis 6, **dadurch gekennzeichnet, dass** das Verhältnis der Dicke der elastischen Folienschicht (6) jeweils zu der Dicke der ersten und/oder der zweiten Deckschicht (4, 5) zwischen 6:1 und 15:1 beträgt.

**8.** Elastische Folie (3) nach einem der Ansprüche 1 bis 7, **dadurch gekennzeichnet, dass** die erste und/oder die zweite Deckschicht (4, 5) aus einem Polyolefin als Hauptbestandteil gebildet sind.

**9.** Elastische Folie (3) nach Anspruch 8, **dadurch gekennzeichnet, dass** die erste und/oder die zweite Deckschicht (4, 5) jeweils ein lineares Polyethylen niedriger Dichte (LLDPE) enthält, wobei der Anteil zumindest 40 Gew.-% beträgt.

**10.** Elastische Folie (3) nach einem der Ansprüche 1 bis 9, **dadurch gekennzeichnet, dass** die erste und/oder die zweite Deckschicht (4, 5) Füllstoffe enthält.

**11.** Elastische Folie (3), insbesondere nach einem der Ansprüche 1 bis 10, mit einer elastischen Folienschicht (6) und zumindest einer ersten, mit der elastischen Folienschicht coextrudierten nicht-elastischen Deckschicht (4, 5), wobei insbesondere die elastische Folienschicht (6) aus einer Polymermischung mit einer ersten Polymerkomponente A und einer zweite Polymerkomponente B gebildet ist, wobei die erste Polymerkomponente ein Polyolefin oder eine Polyolefinmischung ist,
**dadurch gekennzeichnet, dass**
ein elastischer Rückstellwert (ERV) nach einer Aktivierung um 300 % mehr als 0,4 beträgt.

**12.** Elastische Folie (3), insbesondere nach einem der Ansprüche 1 bis 11, mit einer elastischen Folienschicht (6) und zumindest einer ersten, mit der elastischen Folienschicht coextrudierten nicht-elastischen Deckschicht (4, 5), wobei insbesondere die elastische Folienschicht (6) aus einer Polymermischung mit einer ersten Polymerkomponente A und einer zweite Polymerkomponente B gebildet ist, wobei die erste Polymerkomponente ein Polyolefin oder eine Polyolefinmischung ist,
**dadurch gekennzeichnet, dass**
die zweite Polymerkomponente B ein Styrol-Ethylen-Ethylen-Propylen-Styrol-Blockcopolymer (SEEPS) ist und der Permanent-Set nach der Aktivierung um 300 % weniger als 40 % beträgt.

**13.** Elastische Folie (3) nach einem der Ansprüche 1 bis 12, **dadurch gekennzeichnet, dass** die Differenzfläche (9) zwischen der Fläche unterhalb der Ladekurve (L) und der Fläche unterhalb der Entlastungskurve (U) nach der Aktivierung um 300 % bei einer Dehnung um 120 % weniger als 90 N/inch% beträgt.

**15.** Elastisches Laminat mit zumindest einer Decklage (1, 2) aus einem Nonwoven und einer damit verbundenen elastischen Folie (3) nach einem der Ansprüche 1 bis 14.

**16.** Elastisches Laminat nach Anspruch 15, **dadurch gekennzeichnet, dass** die elastische Folie (3) zwischen zwei Decklagen (1, 2) aus einem Nonwoven angeordnet und mit diesen verbunden ist.

**17.** Elastisches Laminat nach Anspruch 15 oder 16, **dadurch gekennzeichnet, dass** die elastische Folie (3) mit der zumindest einen Decklage (1, 2) durch Ultraschallsachweißen verbunden ist.

**18.** Elastisches Laminat nach einem der Ansprüche 15 bis 17, **dadurch gekennzeichnet, dass** die elastische Folie (3) in einem aktivierten Zustand mit der zumindest einen Decklage (1, 2) verbunden ist.

**19.** Hygieneartikel aus einem elastischen Laminat nach einem der Ansprüche 1 bis 18.
